# EUROPÄISCHE PATENTSCHRIFT

(11) **EP 3 846 697 B1**
(45) Veröffentlichungstag und Bekanntmachung des Hinweises auf die Patenterteilung: **31.07.2024**
(21) Anmeldenummer: 19787159.3
(22) Anmeldetag: 04.09.2019
(51) Int. Cl.: A61B 17/00, A61B 17/34, A61M 11/02, A61M 13/00

(54) **MEDIZINISCHES INSTRUMENT ZUM GERICHTETEN EINBRINGEN EINER THERAPEUTISCHEN SUBSTANZ IN EINEN HOHLRAUM UND WERKZEUG DAFÜR**
MEDICAL INSTRUMENT FOR INTRODUCING A THERAPEUTIC SUBSTANCE INTO A CAVITY IN A TARGETED MANNER, AND TOOL FOR SAME
INSTRUMENT CHIRURGICAL DESTINÉ À INTRODUIRE DE FAÇON ORIENTÉE UNE SUBSTANCE THÉRAPEUTIQUE DANS UNE CAVITÉ ET OUTIL S'Y RAPPORTANT

(30) Priorität: 04.09.2018 DE 102018121496
(43) Veröffentlichungstag der Anmeldung: 14.07.2021
(73) Patentinhaber: Prof. Reymond & Hetzel GbR, 78667 Villingendorf (DE)
(72) Erfinder: REYMOND, Marc, 72076 Tübingen (DE); HETZEL, Alexander, 78667 Vilingendorf (DE)
(74) Vertreter: Drobnik, Stefanie
(86) Internationale Anmeldenummer: PCT/EP2019/000254
(87) Internationale Veröffentlichungsnummer: WO 2020/048627

(56) Entgegenhaltungen:
- EP-A1- 2 695 626
- EP-A1- 3 354 213
- WO-A1-2013/046710
- US-A1- 2011 213 296

## Beschreibung

Die Erfindung betrifft ein medizinisches Instrument zum gerichteten Einbringen einer therapeutischen Substanz in einen Hohlraum eines Körpers.

Aus dem Stand der Technik sind medizinische Instrumente zum gerichteten Einbringen einer Substanz in einen Hohlraum eines Körpers bekannt, die in der minimal-invasiven Chirurgie eingesetzt werden. Aus der DE 10 2012 104 629 A1 ist ein solches Instrument bekannt, wobei ein dafür passendes Werkzeug einen Schaft aufweist, an dessen distalem Ende eine Düse angeordnet ist, mit der eine therapeutische Substanz in einem Pneumoperitoneum versprüht werden kann. Die wesentlichen Bestandteile des Werkzeugs sind aus Metall, um es desinfizieren zu können. Zudem weist es einen komplexen inneren Aufbau auf.

Ferner beschreibt EP 3 354 213 A1 ein solches Instrument als Applikator, der eine Trokarhülse aufweist, in der eine Nadel geführt ist. Am distalen Ende der Nadel ist eine Nadeldüse lösbar angebracht, durch deren Öffnungen eine Substanz in den Körperhohlraum eingebracht wird. Ein Schlauch verbindet eine Substanzquelle mit der Trokarhülse.

DE 295 16 077 U1 schlägt eine Sprühvorrichtung für Zwei-Komponenten-Kleber vor, wobei zwei Rohre innerhalb eines steifen Rohrteils verlaufen und distal in einen auf das Rohrteil aufschraubbaren Düsenkopf münden, in dem die Komponenten vor dem Versprühen vermischt werden. Zur separierten Zufuhr der Komponenten sind Rohrstücke am proximalen Ende des Rohrteils zur Verbindung mit einer Doppelspritze angeordnet. Das Dokument US2011/213296 A1 offenbart ein medizinisches Instrument zum Einbringen einer therapeutischen Substanz in einen Hohlraum des Körpers aus dem Stand der Technik.

Ausgehend von diesem Stand der Technik ist es Aufgabe der vorliegenden Erfindung, ein möglichst einfach aufgebautes und kostengünstig herzustellendes medizinisches Instrument zum gerichteten Einbringen einer Substanz in einen Hohlraum eines Körpers ausgebildet ist, bereitzustellen.

Diese Aufgabe wird durch ein medizinisches Instrument mit den Merkmalen des Anspruchs 1 gelöst.

Bevorzugte Ausführungsformen des medizinischen Instruments sind in den Unteransprüchen ausgeführt.

Eine erste Ausführungsform des medizinischen Instruments ist ausgebildet zum gerichteten Einbringen einer therapeutischen Substanz in einen Hohlraum eines Körpers. Das medizinische Instrument weist einen Trokar, eine Fluidquelle, eine Fluidpumpe sowie ein Werkzeug auf, das mit der Fluidquelle und der Fluidpumpe fluidisch verbunden werden kann und über den Trokar in eine Körperhöhle eingeführt werden kann. Das Werkzeug hat einen Schaft mit einem Lumen und weist ferner eine flexible Fluidleitung und einen Düsenkopf auf, wobei die flexible Fluidleitung sich in dem Lumen des Schafts erstreckt und der Düsenkopf mit der flexiblen Fluidleitung verbunden ist. Das proximale Ende der flexiblen Fluidleitung kann mit der Fluidquelle direkt verbunden werden, so dass das Werkzeug mit der Fluidquelle und der Fluidpumpe über die flexible Fluidleitung verbunden ist.

So kann ein geeigneter Fluidstrom von Fluidquelle bis zur Verteilung des Fluids in der Körperhöhle erzeugt werden. Die Fluidquelle kann eine vorbestimmte Substanz, bspw. ein Medikament, ferner ein Gas oder eine Kombination aus beidem beherbergen bzw. noch weitere Komponenten umfassen, die notwendig sind, um die Substanz in die Körperhöhle einzubringen. Eine Körperhöhle kann jeder körpereigene Hohlraum sein, und auch als chirurgisch erzeugter Hohlraum, wie ein künstlich angelegtes Pneumoperitoneum, verstanden werden.

Vorteilhaft wird so mit einem einfachen Werkzeug als Bauteil ein sehr einfach aufgebautes und zuverlässiges medizinisches Instrument geschaffen, bei dem zur fluidischen Überführung der therapeutischen Substanz zum Düsenkopf nur eine Verbindungsstelle, nämlich zwischen der flexiblen Fluidleitung des Werkzeugs und der Fluidquelle mit Fluidpumpe, nötig ist. Somit werden weitere Verbindungsstellen vermieden, insbesondere zwischen einer an die Fluidquelle fluidisch angeschlossenen Fluidzuführleitung bzw. einem Übergangsstück und dem Werkzeug, die gemäß Stand der Technik üblicherweise an dem proximalen Ende des Werkzeugs vorliegt, das dem proximalen Ende des Schafts entspricht. Dadurch liegen weniger Fehlerquellen vor, die beispielsweise Undichtheit und damit Druckverlust in der Leitung bedeuten könnten. Sofern damit weniger Verwendungsmöglichkeiten und ggf. schlechtere Reinigbarkeit des Werkzeugs und damit des medizinischen Instruments einhergehen, ist dies unproblematisch, da das Werkzeug kostengünstig auch als Einmalprodukt verwendbar ist. Die Herstellung des medizinischen Instruments ist zudem kostengünstig. Als Fluid kann eine Flüssigkeit oder ein Gas oder eine Kombination daraus verwendet werden, wobei das Fluid die therapeutische Substanz trägt oder umfasst und mit dem Düsenkopf versprüht werden kann.

Die flexible Fluidleitung des medizinischen Instruments erstreckt sich dabei zumindest über die gesamte Länge des Schafts, bevorzugt aber auch flexibel darüber hinaus, sodass die flexible Fluidleitung direkt mit einer Fluidquelle, in der die therapeutische Substanz vorgelegt ist, verbunden werden kann. Es wird daher ein direkter Anschluss der flexiblen Fluidleitung an die Fluidquelle vorgenommen, es ist keine zusätzliche Fluidleitung, die die therapeutische Substanz von der Quelle zu dem Schaft führt, nötig, und damit auch kein zusätzlicher Anschluss bzw. Verbindungsstücke.

Auch über das distale Ende des Schafts hinaus kann sich die flexible Fluidleitung erstrecken.

In noch einer weiteren Ausführungsform des erfindungsgemäßen medizinischen Instruments ist der Düsenkopf an einem distalen Ende des Werkzeugs angeordnet. Er kann sich teilweise oder vollständig in das Lumen des Schafts erstrecken und wird dadurch bereits stabil positioniert. Bevorzugt ist der Düsenkopf mit der flexiblen Fluidleitung über ein Verbindungsstück verbunden. Alternativ kann der Düsenkopf mit der flexiblen Fluidleitung auch direkt verbunden sein. Die Verbindung kann unlösbar gestaltet sein oder auch als lösbare Verbindung über eine Schraub- oder Bajonettverbindung oder eine andere Verbindungsart ausgebildet sein. Vorteilhaft ist es dadurch nicht zwingend nötig, den Düsenkopf mit dem Schaft zu verbinden, sodass der Schaft sehr einfach gefertigt werden kann.

Eine weitere Ausführungsform des erfindungsgemäßen medizinischen Instruments sieht vor, dass der Schaft aus einem Kunststoff besteht, wobei der Schaft zur Herstellung einer Montageanordnung als vorgefertigtes Rohr über die flexible Fluidleitung geführt werden kann bzw. auch umgekehrt die Fluidleitung in den Schaft geführt werden kann. Alternativ kann der Schaft auch aus dem Kunststoff um die flexible Fluidleitung gespritzt werden und ist damit angepasst an die flexible Fluidleitung. Beim Aufspritzen können zugleich andere Komponenten befestigt werden oder Befestigungselemente vorgesehen werden.

Damit wird eine kostengünstige Herstellung ermöglicht. Bei der Schaftfertigung müssen Form, Größe und Verlauf der flexiblen Fluidleitung nicht berücksichtigt werden, er muss auch keine Positioniermittel für die flexible Fluidleitung enthalten. Es sind keine zusätzlichen Anschlüsse zwischen einer extern von der Fluidquelle kommenden Fluidleitung und Werkzeug-Schaft mehr notwendig, sondern die flexible Fluidleitung ist direkt in den Schaft hinein, durch den Schaft hindurch bis zur Düse selbst geführt.

Die Ausführung aus Kunststoff macht das medizinische Instrument nicht nur kostengünstig in der Herstellung, sondern es kann auch als Einmal-Werkzeug dienen, dass nach einmaligem Gebrauch weggeworfen werden kann. Dadurch, dass es aus wenigen Bestandteilen besteht, ist es auch recycelbar bzw. einfach zu entsorgen.

Ferner können die flexible Fluidleitung und das Verbindungsstück lösbar miteinander verbunden sein. Je nach Ausgestaltung kann die Verbindung über ein Gewinde zwischen beiden Komponenten oder mittels einfachem Stecken realisiert sein. Alternativ können die flexible Fluidleitung und das Verbindungsstück vor Montage aber auch lose vorliegen und zur Herstellung einer Montageanordnung Stoß an Stoß gelegt werden, um dann miteinander verklebt oder mit Kunststoff umspritzt zu werden.

In noch einer weiteren Ausführungsform des erfindungsgemäßen medizinischen Instruments sind die flexible Fluidleitung und das Verbindungsstück, also beide Komponenten miteinander, unlösbar miteinander verbunden. Je nach Ausgestaltung kann die Verbindung zwischen beiden Komponenten beispielsweise mittels Verkleben oder Verschwei-βen realisiert sein.

Um die Komponenten Verbindungsstück und Schaft miteinander zu verbinden, sodass der Düsenkörper, der fest in dem Verbindungsstück aufgenommen ist, fest gefasst werden kann, können der Schaft und das Verbindungsstück zueinander korrespondierende Rastelemente aufweisen, sodass in einer Montageanordnung die Rastelemente im Eingriff zueinander stehen können. Für eine gute Prozesssicherheit können beide Komponenten zueinander korrespondierende Gewinde aufweisen, sodass Verbindungsstück und Schaft miteinander verschraubt werden können.

Ferner kann das medizinische Instrument an seinem distalen Ende eine Schutzkappe aufweisen, die den Düsenkörper umgibt. Das Gleiche wie für die Verbindung Verbindungsstück und Schaft kann für die Schutzkappe realisiert sein, damit diese nicht einfach aufgesteckt, sondern fest am Schaft gehalten werden kann. Die Schutzkappe kann mittels geeigneter Rastelemente mit dem Schaft verrastet werden, der dazu korrespondierende Rastelemente aufweist. Ferner kann die Schutzkappe mit dem Schaft verklebt, verschweißt oder aufgespritzt werden.

Damit das Fluid, das die therapeutische Substanz trägt oder umfasst, auch mittels des Düsenkopfes versprüht werden kann, weist die flexible Fluidleitung ein Lumen auf, in dem das Fluid strömen kann. Die Düse ist ausgebildet zur Erzeugung eines Aerosols und umfasst einen Düsenkörper, der mit dem Verbindungsstück verbunden werden kann. Dazu können in einer weiteren Ausführungsform des medizinischen Instruments beide Komponenten miteinander lösbar verbunden, bevorzugt verschraubt sein. Dazu kann das Verbindungsstück ein Gewinde, bevorzugt ein Innengewinde, aufweisen, wozu der Düsenkörper ein entsprechend korrespondierendes Gegengewinde, bevorzugt ein Außengewinde, aufweisen kann. Das Verbindungsstück hat ferner ein Lumen, das in Strömungsrichtung von einer Durchtrittsbohrung gefolgt wird, die enger als das erste Lumen ist. Die Durchtrittsbohrung liegt in der Montageanordnung benachbart zu einer mittigen Bohrung des Düsenkörpers an und mündet in Strömungsrichtung in einen Zentrierkonus, der das Fluid auf die Düsenbohrung beaufschlagt. Diese ist im Vergleich zu den vorgenannten Lumina dünn ausgebildet, um das Fluid mit Druck zu beaufschlagen und ihm eine entsprechend hohe Strömungsgeschwindigkeit zu verleihen. Aus einer Düsenöffnung, die an der Stirnseite (distales Ende des Werkzeugs) des Düsenkörpers angeordnet ist, kann dann das Fluid ausströmen. Alle genannten Lumina stehen in fluidischer Verbindung, um die therapeutische Substanz zur Düsenöffnung zu führen, die dort versprüht werden soll.

In noch einer weiteren Ausführungsform des medizinischen Instruments weist die flexible Fluidleitung, um das Lumen des Werkzeugs mit einer Fluidquelle und einer Fluidpumpe fluidisch verbinden zu können, einen Anschluss auf, der dazu ausgebildet ist, die Fluidleitung und somit das Werkzeug lösbar mit der Fluidquelle zu verbinden. Bevorzugt handelt es sich um einen medizinisch zugelassenen Standard-Anschluss, besonders bevorzugt um einen Luer-Lock-Anschluss.

Das erfindungsgemäße medizinische Instrument erlaubt, ein sehr einfach aufgebautes Mittel für die minimal-invasive Chirurgie bereitzustellen.

Weitere Ausführungsformen des medizinischen Instruments sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Die Figuren sind lediglich eine schematische Darstellung einer Ausführungsform der Erfindung.

Dabei zeigen:
- **Fig. 1**: eine schematische Ansicht des Werkzeugs,
- **Fig. 2**: einen Längsschnitt nach Schnitt A-A durch das Werkzeug,
- **Fig. 3**: eine Detailansicht B eines distalen Endabschnitts des Werkzeugs, und
- **Fig. 4**: eine schematische Ansicht des erfindungsgemäßen medizinischen Instruments.

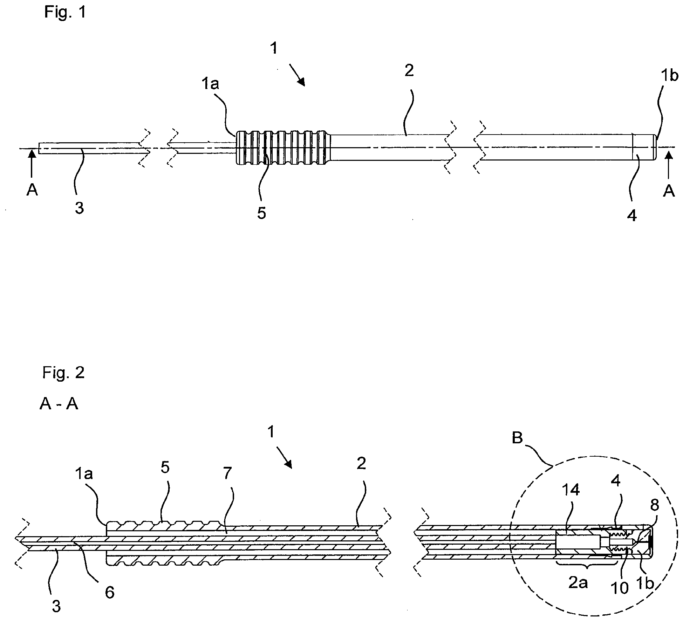

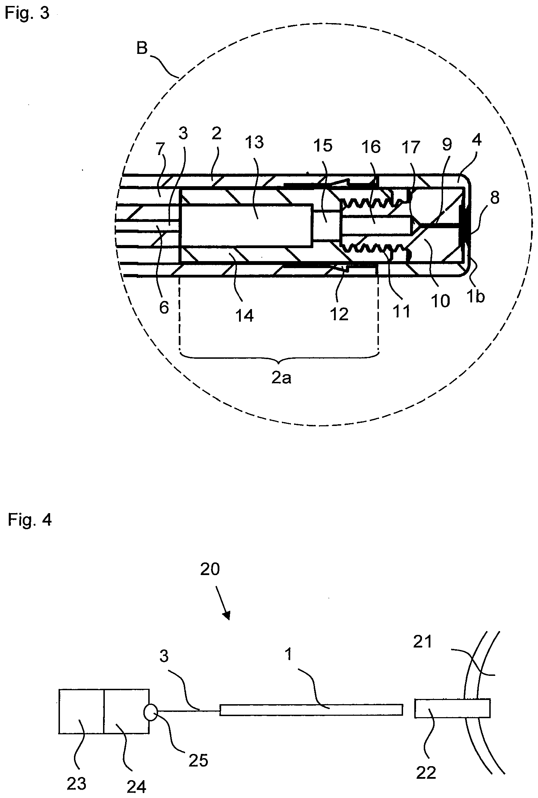

In **Fig. 1** zeigt das Werkzeug 1 einen Schaft 2, der an einem distalen Ende 1b des Werkzeugs 1 mit einer Schutzkappe 4 abschließt. In einem Abschnitt, der sich an ein proximales Ende 1a des Werkzeugs 1 anschließt, ist ein geriffelter Griff 5 an den Schaft 2 ausgebildet. Aus dem proximalen Ende 1a erstreckt sich eine flexible Fluidleitung 3, die mit weiteren Komponenten verbunden ist, um ein medizinisches Instrument 20, wie in **Fig. 4** dargestellt, zu schaffen. Der Schaft 2 ist im Wesentlichen aus einem festen Kunststoff gefertigt, und dient der Stabilisierung der flexiblen Fluidleitung 3.

Das erfindungsgemäße medizinische Instrument 20 in **Fig. 4** ist dazu ausgebildet, mit einer Körperhöhle 21 zusammen zu wirken. Dazu ist in die Körperhöhle 21 ein Trokar 22 eingeführt. Das Werkzeug 1 kann mit diesem Trokar 22 wechselwirken; also in diesen eingeführt werden. Ferner weist das medizinische Instrument 20 eine Fluidquelle 23 auf, die mit einer Fluidpumpe 24 verbunden ist. Über einen Anschluss 25, der insbesondere ein Luer-Lock-Anschluss sein kann, ist die Fluidquelle 23 mit der flexiblen Fluidleitung 3 und damit direkt mit dem Werkzeug 1 lösbar verbunden.

In **Fig. 2** ist ein Schnitt A - A durch das Werkzeug 1 nach **Fig. 1** gezeigt. Ein Ausschnitt B - im Detail in **Fig. 3** gezeigt - zeigt einen Endabschnitt 2a des Werkzeugs 1 aus **Fig. 2****.** Die flexible Fluidleitung 3 hat, wie in dem Längsschnitt in **Fig. 2** gezeigt, ein Lumen 6, durch das das Fluid strömen kann. Die flexible Fluidleitung 3 liegt dabei mit einem länglichen Abschnitt innerhalb des Schafts 2, der dazu ein Lumen 7 aufweist, um eben die flexible Fluidleitung 3 zu hausen, wobei sich die flexible Fluidleitung 3 proximal über das Ende des Schafts 2 hinaus aus dem Schaft 2 heraus erstreckt.

Die flexible Fluidleitung 3 ist durch das Lumen 7 bis zu dem distalen Endabschnitt 2a geführt, in dem ein Verbindungsstück 14 in dem Lumen 7 des Schafts 2 vorliegt. Dieses Verbindungsstück 14 ist zylindrisch und weist ein Lumen 13 auf, dessen Innendurchmesser im Wesentlichen dem Außendurchmesser der flexiblen Fluidleitung 3 entspricht. Verbindungsstück 14 und Fluidleitung 3 sind in **Fig. 2** ineinander gesteckt.

Ferner ist in dem Verbindungsstück 14 eine Durchtrittsöffnung bzw. -Bohrung 15 vorgesehen, die mit dem Lumen 6 der Fluidleitung 3 fluidisch in Verbindung steht, sowie ein Gewinde 11, das sich an die Durchtrittsbohrung 15 anschließt, diese im Durchmesser wieder erweitert und sich in Richtung des distalen Endes 1b des Werkzeugs 1 erstreckt. In das Gewinde 11 eingeschraubt ist ein Düsenkörper 10, der von der Schutzkappe 4 umgeben wird. Der Düsenkörper 10 weist ein zu dem Gewinde 11 korrespondierendes Gewinde auf, sodass der Düsenkörper 10 in das Verbindungsstück 14 eingeschraubt und bei Bedarf ausgetauscht werden kann. Der Düsenkörper 10 weist eine mittige Bohrung 16 auf, die in einen Zentrierkonus 17 mündet und sich schließlich in eine Düsenbohrung 9 erstreckt. An diese Düsenbohrung 9 schließt sich eine Düsenöffnung 8 an, die an der Stirnseite des Werkzeugs 1, also dem distalen Ende 1b des Werkzeugs 1 vorliegt.

Somit wird eine direkte Zuleitung des Fluids zum Düsenkörper 10 über die flexible Fluidleitung 3 realisiert; es ist nicht nötig, das Fluid von einer Fluid führenden Komponente in die nächste zu überführen, sondern die Fluidleitung 3 stellt die komplette Fluidführung von der Fluidquelle 23 bis zum Düsenkörper 10 bereit. Die Verbindung über das Verbindungsstück 14 ist dabei optional; alternativ kann der Düsenkörper 10 direkt, ohne Verbindungsstück, lösbar oder unlösbar, mit der flexiblen Fluidleitung 3 verbunden sein.

Die Schutzkappe 4 wird innerhalb des Schafts 2 mittels Rastelementen 12 gehalten. Diese Rastelemente 12 können sowohl an der Außenseite der Schutzkappe 4 sowie an der Innenseite des Lumens 7 des Schafts 2 ausgebildet sein.

### BEZUGSZEICHENLISTE

- 1: Werkzeug
- 1a: Proximales Ende
- 1b: Distales Ende
- 2: Schaft
- 2a: Distaler Endabschnitt
- 3: Flexible Fluidleitung
- 4: Schutzkappe
- 5: Griff
- 6: Lumen Fluidleitung
- 7: Lumen Schaft
- 8: Düsenöffnung
- 9: Düsenbohrung
- 10: Düsenkörper
- 11: Gewinde
- 12: Rastelemente
- 13: Lumen Verbindungsstück
- 14: Verbindungsstück
- 15: Durchtrittsbohrung
- 16: Mittige Bohrung
- 17: Zentrierkonus
- 20: Medizinisches Instrument
- 21: Körperhöhle
- 22: Trokar
- 23: Fluidquelle
- 24: Fluidpumpe
- 25: Anschluss

## Patentansprüche

1. Medizinisches Instrument (20) zum Einbringen einer therapeutischen Substanz in eine Körperhöhle (21), mit einem Trokar (22), einer Fluidquelle (23), einer Fluidpumpe (24) sowie einem Werkzeug (1), das mit der Fluidquelle (23) und der Fluidpumpe (24) fluidisch verbindbar ist und über den Trokar (22) in eine Körperhöhle (21) einführbar ist, wobei das Werkzeug (1) einen starren Schaft (2) mit einem Lumen (7) aufweist und eine flexible Fluidleitung (3) und einen Düsenkopf (10) aufweist, wobei die flexible Fluidleitung (3) sich in dem Lumen (7) des starren Schafts (2) erstreckt und der Düsenkopf (10) mit der flexiblen Fluidleitung (3) verbunden ist, wobei das proximale Ende der flexiblen Fluidleitung (3) mit der Fluidquelle (23) direkt verbindbar ist, und die Verbindung des Werkzeugs (1) mit der Fluidquelle (23) und der Fluidpumpe (24) bereitstellt, und wobei der Düsenkopf (10) des Werkzeugs (1) an einem distalen Ende (1 b) des Werkzeugs (1) angeordnet ist und sich teilweise oder vollständig in das Lumen (7) des starren Schafts (2) erstreckt; und der Düsenkopf (10) mit der flexiblen Fluidleitung (3) direkt oder über ein Verbindungsstück (14) verbunden ist.

2. Medizinisches Instrument (20) nach Anspruch 1,
**dadurch gekennzeichnet, dass**
der Schaft (2) aus einem Kunststoff besteht, wobei der Schaft (2) zur Herstellung einer Montageanordnung als vorgefertigtes Rohr über die flexible Fluidleitung (3) führbar ist oder aus dem Kunststoff um die flexible Fluidleitung (3) spritzbar ist.

3. Medizinisches Instrument (20) nach zumindest einem der Ansprüche 1 bis 2,
**dadurch gekennzeichnet, dass**
die flexible Fluidleitung (3) und das Verbindungsstück (14) unlösbar miteinander verbunden sind.

4. Medizinisches Instrument (20) nach zumindest einem der Ansprüche 1 bis 3,
**dadurch gekennzeichnet, dass**
der Schaft (2) und das Verbindungsstück (14) zueinander korrespondierende Rastelemente (12) aufweisen, so dass in einer Montageanordnung die Rastelemente (12) im Eingriff zueinander sind.

5. Medizinisches Instrument (20) nach zumindest einem der Ansprüche 1 bis 4,
**dadurch gekennzeichnet, dass**
das Werkzeug (1) an seinem distalen Ende (1a) eine Schutzkappe (4) aufweist, die den Düsenkörper (10) umgibt.

6. Medizinisches Instrument (20) nach zumindest einem der Ansprüche 1 bis 5,
**dadurch gekennzeichnet, dass**
die flexible Fluidleitung (3) ein Lumen (6) aufweist, in dem das Fluid strömbar ist.

7. Medizinisches Instrument (20) nach zumindest einem der Ansprüche 1 bis 6,
**dadurch gekennzeichnet, dass**
die flexible Fluidleitung (3) zur fluidischen Verbindung des Lumens (6) mit einer Fluidquelle (23) und der Fluidpumpe (24) einen Anschluss (25) aufweist, wobei der Anschluss (25) ein Luer-Lock-Anschluss ist.

## Claims

1. A medical instrument (20) for introducing a therapeutic substance into a body cavity (21), having a trocar (22), a fluid source (23), a fluid pump (24) and a tool (1) which can be fluidically connected to the fluid source (23) and the fluid pump (24) and can be introduced into a body cavity (21) via the trocar (22),
wherein the tool (1) has a rigid shaft (2) with a lumen (7) and has a flexible fluid line (3) and a nozzle head (10), wherein the flexible fluid line (3) extends in the lumen (7) of the rigid shaft (2) and the nozzle head (10) is connected to the flexible fluid line (3), wherein the proximal end of the flexible fluid line (3) is directly connectable to the fluid source (23) and provides the connection of the tool (1) to the fluid source (23) and the fluid pump (24), and wherein
the nozzle head (10) of the tool (1) is arranged at a distal end (1b) of the tool (1) and extends partially or completely into the lumen (7) of the rigid shaft (2); and the nozzle head (10) is connected to the flexible fluid line (3) directly or via a connecting piece (14).

2. Medical instrument (20) according to claim 1,
**characterised in that**
the shaft (2) is made of a plastic, wherein the shaft (2) can be guided over the flexible fluid line (3) as a prefabricated tube to produce an assembly arrangement or can be injection-moulded from the plastic around the flexible fluid line (3).

3. Medical instrument (20) according to at least one of claims 1 to 2,
**characterised in that**
the flexible fluid line (3) and the connecting piece (14) are non-detachably connected to each other.

4. Medical instrument (20) according to at least one of claims 1 to 3,
**characterised in that**
the shaft (2) and the connecting piece (14) have corresponding latching elements (12), so that in an assembly arrangement the latching elements (12) are in engagement with one another.

5. Medical instrument (20) according to at least one of claims 1 to 4,
**characterised in that**
the tool (1) has a protective cap (4) at its distal end (1a), which surrounds the nozzle body (10).

6. Medical instrument (20) according to at least one of claims 1 to 5,
**characterised in that**
the flexible fluid line (3) has a lumen (6) in which the fluid can flow.

7. Medical instrument (20) according to at least one of claims 1 to 6,
**characterised in that**
the flexible fluid line (3) has a connector (25) for fluidic connection of the lumen (6) to a fluid source (23) and the fluid pump (24), wherein the connector (25) is a Luer-lock-connector.

## Revendications

1. Instrument médical (20) destiné à l'introduction d'une substance thérapeutique dans une cavité corporelle (21), comprenant un trocart (22), une source de fluide (23), une pompe à fluide (24) ainsi qu'un outil (1) qui peut être relié fluidiquement à la source de fluide (23) et à la pompe à fluide (24) et qui peut être introduit dans une cavité corporelle (21) par le biais du trocart (22),
**dans lequel**
l'outil (1) possède une tige rigide (2) pourvue d'une lumière (7) et possède une conduite de fluide flexible (3) et une tête de buse (10), la conduite de fluide flexible (3) s'étendant dans la lumière (7) de la tige rigide (2) et la tête de buse (10) étant connectée à la conduite de fluide flexible (3), dans lequel l'extrémité proximale de la conduite de fluide flexible (3) peut être directement connectée à la source de fluide (23) et fournit la connexion de l'outil (1) à la source de fluide (23) et à la pompe de fluide (24), et dans lequel
la tête de buse (10) de l'outil (1) est disposée à une extrémité distale (1b) de l'outil (1) et s'étend partiellement ou complètement dans la lumière (7) de la tige rigide (2) ; et la tête de buse (10) est reliée à la conduite de fluide flexible (3) directement ou par l'intermédiaire d'une pièce de raccordement (14).

2. Instrument médical (20) selon la revendication 1,
**caractérisé en ce que**
la tige (2) est constituée d'une matière plastique, la tige (2) pouvant être guidée sur la conduite de fluide flexible (3) en tant que tube préfabriqué pour la fabrication d'un agencement de montage ou pouvant être projetée à partir de la matière plastique autour de la conduite de fluide flexible (3).

3. Instrument médical (20) selon au moins l'une des revendications 1 à 2,
**caractérisé en ce que**
la conduite de fluide flexible (3) et la pièce de raccordement (14) sont reliées l'une à l'autre de manière indissociable.

4. Instrument médical (20) selon au moins l'une des revendications 1 à 3,
**caractérisé en ce que**
la tige (2) et la pièce de raccordement (14) possèdent des éléments d'encliquetage (12) correspondant les uns aux autres, de sorte que dans un agencement de montage, les éléments d'encliquetage (12) sont en prise les uns avec les autres.

5. Instrument médical (20) selon au moins l'une des revendications 1 à 4,
**caractérisé en ce que**
l'outil (1) possède, à son extrémité distale (1a), un capuchon de protection (4) qui entoure le corps de buse (10).

6. Instrument médical (20) selon au moins l'une des revendications 1 à 5,
**caractérisé en ce que**
la conduite de fluide flexible (3) possède une lumière (6) dans laquelle le fluide peut s'écouler.

7. Instrument médical (20) selon au moins l'une des revendications 1 à 6,
**caractérisé en ce que**
la conduite de fluide flexible (3) possède un raccord (25) destiné à la liaison fluidique de la lumière (6) avec une source de fluide (23) et la pompe à fluide (24), le raccord (25) étant un raccord Luer-Lock.
